Europäisches Patentamt

European Patent Office

Office européen des brevets

(19)

(11) Publication number : **0 469 281 B1**

# (12) EUROPEAN PATENT SPECIFICATION

(45) Date of publication of patent specification :
16.06.93 Bulletin 93/24

(51) Int. Cl.⁵ : **C07K 7/08,** C07K 7/10,
A61K 39/29, A61K 39/295

(21) Application number : 91110233.3

(22) Date of filing : 21.06.91

(54) Amino acid residue sequence of hepatitis B core antigen.

(30) Priority : 31.07.90 GB 9016727

(43) Date of publication of application :
05.02.92 Bulletin 92/06

(45) Publication of the grant of the patent :
16.06.93 Bulletin 93/24

(84) Designated Contracting States :
AT BE CH DE DK ES FR GB GR IT LI LU NL SE

(56) References cited :
J. Clin, Invest. 88, 214-222 (july '91)

(73) Proprietor : CLONIT S.p.A
Via Ercole Ferrario, 6
I-20144 Milano (IT)

(72) Inventor : Ferrari, Carlo
Via Corelli 1
I-43100 Parma (IT)
Inventor : Colucci, Giuseppe
Via Santa Maria Fulcorina 20
I-20123 Milan (IT)

(74) Representative : Bellenghi, Mario Dr. et al
Ing. A. Giambrocono & C. s.r.l. Via Rosolino
Pilo, 19/B
I-20129 Milano (IT)

## Description

## AMINO ACID RESIDUE SEQUENCE OF HEPATITIS B CORE ANTIGEN

The present invention relates to a T cell stimulating peptide consisting of an amino acid residue sequence of formula (I)

$$\text{PHHTALRQAILCWGELMTLA} \qquad \text{(I)}$$

useful in a diagnostic kit for and in a vaccine against hepatitis B virus.

It is known that anti-envelope antibodies can neutralize viral particles and therefore can confer protection against hepatitis B virus (hereinafter HBV) infection. Based on this evidence, vaccines consisting of hepatitis B surface antigen (hereinafter HBsAg) purified from chronic HBsAg carriers or produced by recombinant DNA technology, have been developed and proved to be highly effective in preventing HBV infection (see WO-A-8810301 and EP-A-354109). An adequate antibody response is usually induced by current HBsAg vaccines in more than 90% of healthy persons, but the proportion of non responder vaccinees is much higher among hemodialysis patients and other immunocompromised persons who, in addition, require an increased number of doses and a larger amount of immunogen to develop a protective immune response.

In an attempt to produce vaccines with enhanced immunogenic potential, inclusion of preS sequences, i.e. entire defined region of the HBsAg protein or peptide having a corresponding amino acid residue sequence, of the HBV envelope in the HBsAg-based vaccine has been proposed. Anyhow it appears that HBsAg may not be effective enough in stimulating a T cell response. The high pathogenetical importance of the T cells is based on the finding that autologous liver cells expressing HBV antigens can be lysed in vitro by peripheral blood T cells and that this phenomenon can be selectively blocked by monoclonal antibodies sensitized by HBsAg. Also it has been recently observed that CD4+ and CD8+ T cells are present in infected liver at the site of cell injury and viral synthesis. Thus the role of T cells is particularly evident in patients with chronic HBV infection: in such patients the in vitro peripheral blood T cell response is dramatically lower than that displayed by patients with acute HBV infection. This difference may have important pathogenetic implications as appearance of a detectable level of T cell sensitization appears to be associated with the clearance of viral particles from sera of subjects with self-limited acute HBV infection. In view of what said the use of other components of HBV for enhancing the HBsAg-based vaccines ability to stimulate a T cell response is desirable, particularly in the case of chronic HBV infection.

On the other hand the HBV nucleocapsid or core antigen (hereinafter HBcAg) represents a good candidate for the development of more immunogenic anti-HBV vaccines because it is a very powerful T cell immunogen. HBcAg is an important sensitizing antigen for intrahepatic T cells during chronic and acute HBV infection and apparently represents a target structure for the immune aggression of infected hepatocytes. The immunization of chimpanzees and woodchucks with HBcAg provides complete or partial protection against HBV infection. Furthermore HBcAg can function as a carrier and adjuvant for other antigens. Obviously HBcAg cannot be used as such in vaccines as it is known that in its complete amino acid sequence some residues have a T cell inhibitory activity that reduces HBcAg effectiveness.

U.S.-A-4,882,145 discloses peptides consisting of T cell stimulating amino acid residue sequences of HBcAg. Particularly said patent discloses the use of the following amino acid residue sequences

| Peptide designation | Amino acid residue sequence |
|---|---|
| p1-20 | MDIDPYKEFGATVELLSFLP |
| p28-52 | RDLLDTASALYREALESPEHCSPHH- |
| p70-94 | TWVGVNLEDPASRDLVVSTVNTNMG |
| p85-100 | VVSYVNTNMGLKFRQL |
| p85-96 | VVSYVNTNMGLK |
| p100-120 | LLWFHISCLTFGRETVIEYLV |
| p100-110 | LLWFHISCLTF |
| p120-140 | VSFGVWIRTPPAYRPPNAPIL |
| p120-131 | VSFGVWIRTPPA |
| p129-140 | PPAYRPPNAPIL |
| p125-136 | WIRTPPAYRPPN |

which have been found to provide a fine specificity for the murine T cell response to HBcAg.

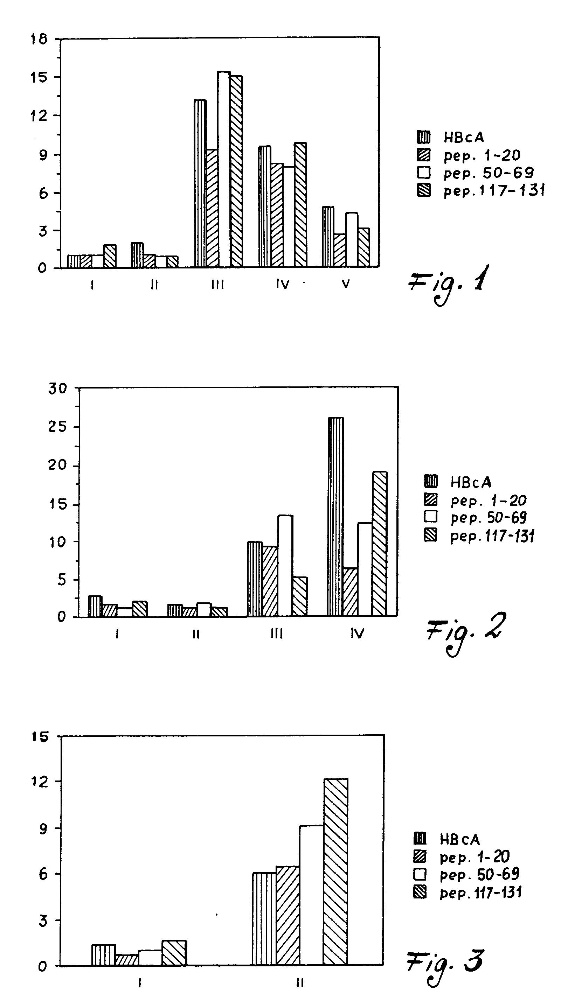

Besides to the above listed preferred sequences, the 32-53, 45-65 (fig.1) and 53-74 sequences have been also therein disclosed

It has been now surprising found that the amino acid residue sequence having formula (I)

PHHTALRQAILCWGELMTLA (I)

and corresponding to position 50-69 from the amino terminus of the HBcAg is able to elicit a T cell response in nearly all the human patients tested.

Accordingly the present invention relates to a T cell stimulating polypeptide consisting essentially of the amino acid residue sequence of formula (I). This T cell stimulating polypeptide is prepared by a process which comprises chemically synthesising the said polypeptide or preparing the said polypeptide by recombinant DNA methodology. The T cell stimulating polypeptide is useful in diagnostic kits for and vaccines against HBV.

The present invention further provides a composite polypeptide immunogen comprising the T cell stimulating polypeptide of formula (I) operatively linked to a polypeptide immunogen. The T cell stimulating polypeptide of formula (I) thus enhances the immunogenicity of the polypeptide immunogen. The invention also provides a process for the preparation of the composite polypeptide immunogen, which process comprises operatively linking the T cell stimulating polypeptide to the polypeptide immunogen.

In the accompanying drawings:

Figs. 1-4 illustrate determinations of the peripheral blood mononuclear proliferative (hereinafter PBMC) response (stimulation index in ordinate) to HBcAg and to the peptide of formula (I) compared to two other T cell stimulating peptides at different times (in abscissa) during the course of acute HBV infection in four representative patients;

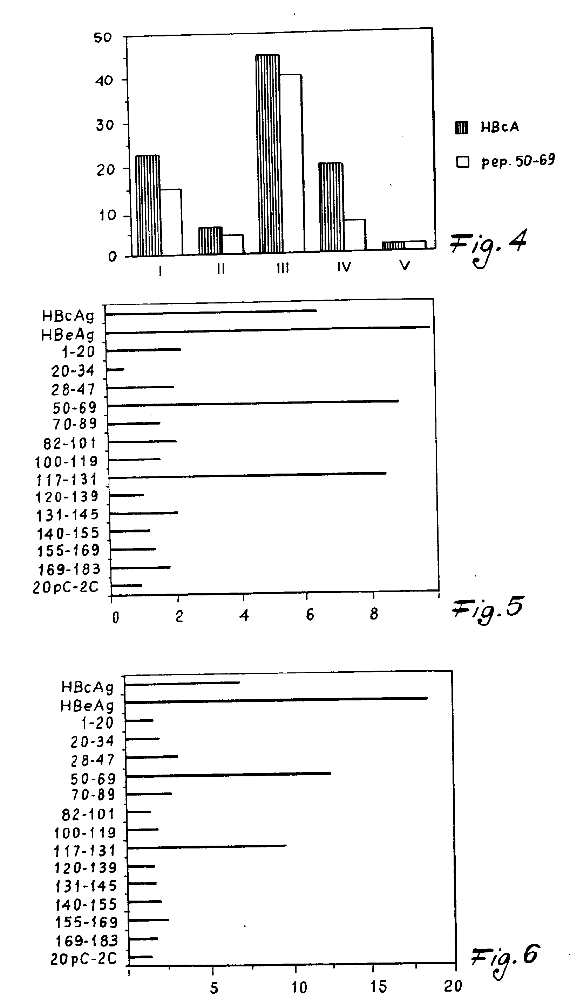

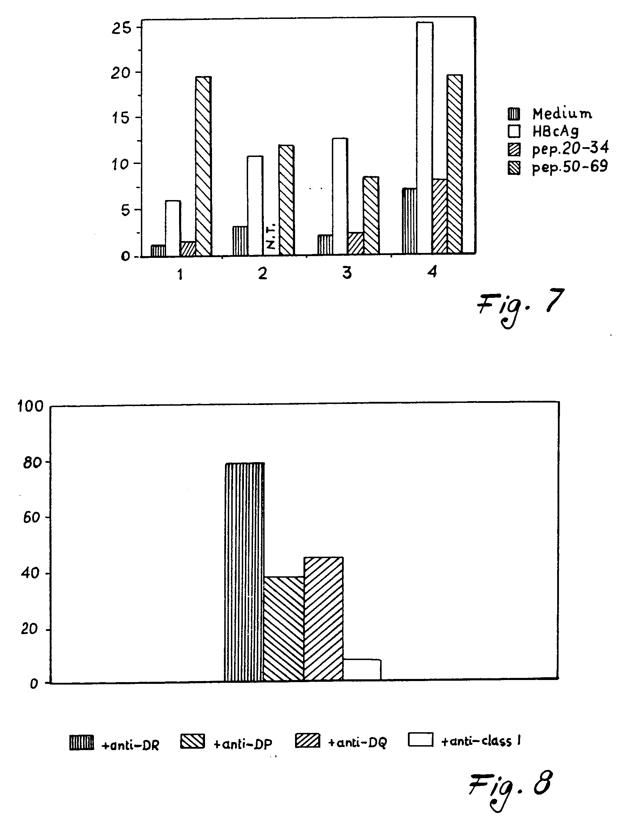

Figs. 5 and 6 illustrate the proliferative response (stimulation index) to HBcAg, HBeAg (i.e. a HBcAg deletion mutant lacking the carboxy-terminal 39 amino acid residues of the core molecule) and synthetic peptides of two representative polyclonal T cell lines produced from peripheral blood T cell stimulated either with HBcAg (Fig. 5) or with HBeAg (Fig. 6);

Fig. 7 illustrates the proliferative response (cpm x 1000 in ordinate) to HBcAg, peptide 50-69 and an irrelevant peptide 20-34 of four representative polyclonal T cell lines (in abscissa) produced from peripheral blood T cell stimulated with peptide 50-69; and

Fig. 8 illustrates the HLA restriction of 50-69 peptide-idnuced T cell proliferation (% inhibition in ordinate). Anti-HLA class I and anti-HLA class II monoclonal antibodies were added, together with the stimulatory peptide, to T cells from a representative peptide-primed polyclonal T cell line specific for 50-69 and $^3$H-thymidine incorporation was measured 3 days later.

The inventors have found an immunodominant T cell peptide (AA-50-69) corresponding to formula (I) within the core molecule which is recognized by more than 95% of patients with acute HBV infection and different HLA haplotypes.

Accordingly, the present T cell peptide may be exploited to enhance the immune reponse to the existing recombinant HBV envelope vaccines and to overcome tolerance to HBsAg particles in non-responder vaccines.

Also, the observation that the peptide 50-69 of formula (I) represents a pure T cell recognition site may be relevant with respect to its inclusion in a synthetic vaccine in the light of the suggested possibility that anti-

EP 0 469 281 B1

HBc antibodies coud inhibit the killing of infected liver cells by masking core epitopes expressed on the hepatocyte surface to the recognition by cytolytic T cells. In addition, the immunodominant T cell peptide of the invention represents a good candidate for a totally synthetic vaccine, i.e. a vaccine not including HBsAg.

The amino acid sequence 50-69 of the invention appears to preferentially activate CD4+ T cells which recognize peptide fragments in the context of HLA class II molecules as shown by blocking experiments with anti-HLA monoclonal antibodies and phenotypic analysis of peptide-primer T cell line. The observation that this peptide is recognized by most HBcAg-responsive patients provides the possibility that it can efficiently bind to many different HLA alleles.

The T cell stimulating polypeptide of formula (I) is operatively linked to a polypeptide immunogen to obtain a composite polypeptide immunogen. Both the T cell stimulating function of the polypeptide of formula (I) and the ability of the polypeptide immunogen to induce antibody that immunoreacts therewith are retained in the composite polypeptide immunogen. Indeed, the presence of the polypeptide of formula (I) enhances the immunogenicity of the polypeptide immunogen.

The polypeptide of formula (I) may be operatively linked through an amino acid residue side chain to the polypeptide immunogen. Heterobifunctional agents are useful which generate a disulfide link at one functional group end and a peptide link at the other, including N-succinimidyl-3-(2-pyridyldithio) propionate (SPDP). This reagent creates a disulfide linkage between itself and a cysteine residue in one protein and an amide linkage through the amino on a lysine or other free amino group in the other. A variety of such disulfide/amide forming agents are known. See for example Immun. Rev. (1982) 62:185. Other bifunctional coupling agents form a thioether rather than a disulfide linkage. Many of these thioether forming agents are commercially available and include reactive esters of 6-maleimidocaproic acid, 2-bromoacetic acid, 2-iodoacetic acid, 4-(N-maleimidomethyl) cyclohexane-1-carboxylic acid and the like. The carboxyl groups can be activated by combining them with succinimide or 1-hydroxy-2-nitro-4-sulfonic acid, sodium salt. A particularly useful coupling agent is succinimidyl 4-(N-maleimidomethyl) cyclohexane-1-carboxylate (SMCC).

The polypeptide of formula (I) may be operatively linked through a peptide bond to the polypeptide immunogen. A polypeptide may therefore be provided whose amino acid sequence comprises both the sequence of the T cell stimulating polypeptide of formula (I) and the sequence of the polypeptide immunogen. Such a fusion protein may be prepared by chemical synthesis or recombinant DNA methodology.

The polypeptide of formula (I) and the polypeptide immunogen may be fused directly to each other. Alternatively, a number of spacer amino acid residues may be provided there between.

Such a spacer group may comprise up to 10, for example up to 5, amino acid residues. The carboxy terminus of the polypeptide of formula (I) may be fused to the amino terminus of the polypeptide immunogen or vice versa. In other words the polypeptide of formula (I) and the polypeptide immunogen are arranged head-to-tail in a fusion protein of the invention.

The polypeptide of formula (I) and the polypeptide immunogen need not be linked together covalently. Another method for operatively linking the T cell stimulating polypeptide of this invention and a pathogen-related polypeptide of interest includes entrapment within an artificial particulate structure, such as, for example, liposomes and ISCOM's (Immunostimulating complex). The use of liposomes and ISCOMS as vehicles for delivering the effective ingredients of a vaccine are well known in the art, as is their preparation. See, for example, Gregoriadis, Trends Biotech, 3:235-241 (1986) and North et al., Proc. Natl. Acad. Sci. U.S.A. 79:7504-08 (1982), for liposome preparation and Morein et al., Nature, 308:457-460 (1987) and Dalsgaard, Arch ges. Virusforsch, 44:243-254 (1974) for ISCOM preparation. A liposome or ISCOM of the present invention thus includes, as effective ingredients, the T cell stimulating polypeptide of the present invention and a pathogen-related polypeptide.

The polypeptide immunogen comprises an antibody-inducing epitope, i.e. a B-cell epitope. This epitope may be an antibody-inducing epitope of a virus, bacterium, fungus or protozoan. Indeed, any polypeptide immunogen against which antibody production is desired can be linked to the T cell stimulating polypeptide of formula (I). In preferred embodiments the polypeptide immunogen is a pathogen-related immunogen and the composite polypeptide immunogen has the capacity to induce the production of antibodies that immunoreact with the pathogen when injected in an effective amount into an animal.

Exemplary immunogens of particular importance are derived from bacteria such as B.pertussis, S. typhosa, S. paratyphoid A and B, C. diphtheriae, C. tetani, C. botulinum C. perfringens, B. anthracis, P. pestis, P. multocida, V. cholerae, N. meningitidis, N.gonorrhee, H.influenzae, T. palladium, and the like; immunogens derived from viruses such as polio virus, adenovirus, parainfluenza virus, measles, mumps, respiratory syncytical virus, influenza virus, equine encephalomyeitis virus, hog cholera virus, Newcastle virus, fowl pox virus, rabies virus, feline and canine distemper viruses, foot-and-mouth disease virus, human and simian immunodeficiency viruses, and the like; rickettsiae immunogen such as epidemic and endemic typhus, and the spotted fever groups, and the like.

4

EP 0 469 281 B1

A useful polypeptide immunogen comprises an epitope of hepatitis B virus, influenza virus, foot-and-mouth disease virus, poliovirus, herpes simplex virus, rabies virus, human immunodeficiency virus or <u>Plasmodium falciparum</u>. A preferred polypeptide immunogen is HBsAg or a polypeptide comprising part of the amino acid sequence of HBsAg. As used herein, HBsAg refers to the naturally occurring filamentous and spherical 22nm particles, the individual major polypeptides and their glycosylated forms that comprise the particles (e.g. p25/gp28, p39/gp42 and gp33/gp36), and synthetic polypeptides that correspond in amino acid sequence to portions of the individual proteins and glycoproteins.

Thus, in one embodiment, the pathogen related immunogen is filamentous or spherical HBsAg. In another embodiment, the pathogen related immunogen is a 33 kilodalton HBsAg protein or a 25 kilodalton HBsAg protein. In another embodiment, the polypeptide immunogen is a synthetic polypeptide that corresponds to a portion of the pre-S region of HBsAg located between an amino-terminal and carboxy-terminal position, respectively, selected from the group consisting of 1-21, 16-27, 32-53, 53-74, 94-105, 94-117, 120-140, 120-145, 128-138, 133-139, 133-140, 133-143, 133-145, 135-143, 135-145, 137-143, 133-151 and 153-171. In yet another embodiment, the polypeptide immunogen corresponds to a portion of the S region of HBsAg located between amino-terminal and carboxy-terminal positions, respectively, selected from 110-137, 117-137, 122-137 and 135-155.

The T cell stimulating polypeptide, fusion proteins and, typically, polypeptide immunogen are synthetic peptides. They may be prepared by chemical synthesis, particularly the shorter such polypeptides. A polypeptide may be built up from single amino acids and/or preformed peptides of two or more amino acid residues in the order of the sequence of the desired polypeptide.

In solid-phase synthesis, the amino acid sequence of the desired polypeptide is built up sequentially from the C-terminal amino acid which is bound to an insoluble resin. When the desired polypeptide has been produced, it is cleaved from the resin. When solution-phase synthesis is employed, the desired polypeptide may again be built up from the C-terminal amino acid. The carboxy group of this acid remains blocked throughout by a suitable protecting group, which is removed at the end of the synthesis.

Whichever technique, solid-phase or solution-phase, is employed each amino acid added to the reaction system typically has a protected amino group and an activated carboxy group. Functional side-chain groups are protected too. After each step in the synthesis, the amino-protecting group is removed. Side-chain functional groups are generally removed at the end of the synthesis.

The polypeptide may also be prepared by recombinant DNA methodologies. Thus, a DNA sequence encoding the polypeptide is provided. An expression vector is prepared which incorporates the DNA sequence and which is capable of expressing the polypeptide when provided in a suitable host. The DNA sequence is located between translation start and stop signals in the vector. Appropriate transcriptional control elements are also provided, in particular a promoter for the DNA sequence and a transcriptional termination site. The DNA sequence is provided in the correct frame such as to enable expression of the peptide to occur in a host compatible with the vector.

Any appropriate host-vector system may be employed. The vector may be a plasmid. In that event, a bacterial or yeast host may be used. Alternatively, the vector may be a viral vector. This may be used to transfect cells of a mammalian cell line in order to cause peptide expression.

The composite polypeptide immunogens can raise neutralising antibody. They therefore may be used as vaccines for humans. Vaccination is achieved by administering to a patient an effective amount of a composite polypeptide immunogen. A human may therefore be vaccinated against a polypeptide immunogen by administering thereto an effective amount of the composite polypeptide immunogen. An oral route or a parenteral route such as sub-cutaneously, intravenously or intramuscularly may be adopted. The dose depends on a variety of factors such as the type of composite polypeptide immunogen, the condition of the patient and the purpose of the treatment. Typically, a composite polypeptide immunogen is administered in an amount of 1 to 1,000 µg per dose, more preferably 10 to 100 µg per dose, by either the oral or the parenteral route. The T cell stimulating polypeptide of formula (I) can be used at a concentration of 0.01 to 100 µg/ml, especially from 1 to 10 µg/ml.

For vaccination purposes, a polypeptide of formula (I) or a composite polypeptide immunogen is typically formulated with a pharmaceutically acceptable carrier or diluent. Conventional formulations, carriers, adjuvants and diluents may be employed. These will of course be determined by the route of administration. Suitable carriers and diluents include Freund's incomplete adjuvant (IFA), aluminium hydroxide, saponin, DEAE-dextran, muramyl dipeptide, mineral oils, neutral oils such as miglycol, vegetable oils such as arachis oil, Pluronic (trade mark) polyols or the Ribi adjuvant system (GB-A-2189141).

The following Example illustrates the invention

EXPERIMENTAL METHODS

1. Peripheral blood mononuclear cell (PBMC) response Test

Twenty-three patients (6 women and 17 men) with acute hepatitis B were studied. The diagnosis was based on the finding of elevated values of serum glutamic pyruvic transaminase (SGPT) activity (at least 10 times the upper level of the normal), associated with the detection of IgM anti-HBc antibodies in the serum and the recent onset of jaundice and other typical symptoms of acute hepatitis. All patients completely recovered from the illness, with normalization of transaminase and clearance of HBsAg from the serum. Control experiments were performed on 13 healthy subject with no evidence of previous exposure of HBV, i.e. they were negative for HBsAg, antibody to HBsAg (anti-Hs) and antibody to HBcAg (anti-HBc).

Said patients with acute hepatitis B were chosen for the study of the fine specificity of the T cell response to HBcAg since a strong level of T cell response to HBcAg and HBeAg is always detectable during the acute phase of HBV infection.

The complete panel of 17 synthetic peptide analogues of HBcAg and HBeAg used in this study is represented in Table 1.

Even though only some peptides had overlapping residues, at least one T cell epitope was identified for each patient. It is remarkable that all patients with various HLA haplo-types except for one (patient 12 of Table 1) showed a significant level of T cell response to the 50-69 sequence. Concentration of this peptide as low as 0.01 µg/ml were stimulatory for peripheral blood T cells of several patients; maximum proliferation was generally reached when peripheral blood T cells were cultured in the presence of 1 to 10 µg/ml of the peptide of the invention. Table 1 reports the PBMC response to HBcAg synthetic peptides of 21 patients with acute hepatitis B. PBMC ( $2 \times 10^5$ cells/well) were cultured in the presence of various peptide concentrations ranging from 0.01 to 100 µg/ml.

## TABLE 1

IIBc-peptides

| Patients' HLA | 1-20 | 20-34 | 28-47 | 50-59 | 50-69 | 70-89 | 02-101 | 100-119 | 117-131 | 120-139 | 131-145 | 140-155 | 155-169 | 169-183 | 20pc-2c |
|---|---|---|---|---|---|---|---|---|---|---|---|---|---|---|---|
| 1 DR1.7.53 DQv1.2 | ++ | - | + | n.t. | + | - | - | n.t. | n.t. | + | n.t. | - | - | - | - |
| 2 DR6.52 DQv1.3 | - | - | - | n.t. | +++ | - | - | - | n.t. | - | n.t | - | - | - | - |
| 3 DR5.6.52 DQv1.3 | - | - | - | n.t. | +++ | - | +++ | + | n.t. | - | n.t. | - | - | - | - |
| 4 DR5.7.52.53 DQv2.3 | + | - | ++ | n.t. | +++ | - | - | - | +++ | - | n.t. | - | - | - | - |
| 5 DR2.5.52 DQv1.3 | ++ | - | + | n.t. | +++ | - | - | - | n.t. | + | n.t. | - | - | - | - |
| 6 DR3.7.52.53 DQ2.3 | +++ | - | +++ | n.t. | +++ | - | - | - | n.t. | + | n.t. | - | - | - | - |
| 7 DR3.5.52 DQv2.3 | +++ | - | + | n.t | +++ | - | - | + | n.t. | - | n.t. | - | - | - | - |
| 8 DR5.52 DQv3 | - | - | - | - | +++ | - | - | +++ | +++ | ++ | - | - | - | - | ++ |
| 9 DR7.53 DQ2 | +++ | ++ | ++ | - | +++ | - | - | - | + | - | - | - | - | - | - |
| 10 DR7.8.53 DQ3.2 | +++ | - | ++ | n.t. | +++ | - | - | - | +++ | - | + | - | + | - | - |
| 11 DR6.11.52 DQv1 | - | - | - | n.t. | +++ | - | - | - | n.t. | - | n.t. | - | - | - | + |
| 12 DR3.11.52 DQv2.3 | +++ | - | - | n.t. | - | - | - | - | n.t | - | n.t. | - | - | - | - |
| 13 DR5.10.52 DQ1.3 | - | - | - | - | +++ | - | + | - | - | - | - | - | - | - | - |
| 14 DR2.6.52 DQ1 | ++ | - | + | - | +++ | - | - | ± | +++ | - | - | - | - | - | - |
| 15 DR3.5.52 DQ2.3 | ++ | - | - | - | ++ | - | - | - | - | - | - | - | - | - | - |
| 16 DR (n.t.) DQ (n.t.) | - | - | - | n.t. | +++ | + | - | - | +++ | - | - | - | - | - | - |
| 17 DR3.52 DQ2 | ++ | - | - | - | ++ | + | - | - | + | + | - | - | - | - | - |
| 18 DR3.6.52 DQ1.2 | - | - | - | - | ++ | - | - | - | - | + | - | - | - | - | - |
| 19 DR (n.t.) DQ (n.t.) | ++ | ++ | - | - | ++ | - | - | + | ++ | + | - | + | - | - | - |
| 20 DR3.52 DQ2 | ++ | - | - | - | ++ | ++ | ++ | ++ | ++ | ++ | - | - | - | - | ++ |
| 21 DR1.2 DQv1 | - | - | +++ | n.t. | +++ | - | +++ | + | +++ | - | - | - | - | +++ | - |

Note:

+++ shows significant proliferative responses obtained with peptide concentrations lower than 1 µg/ml;

++ means significant proliferative responses obtained with peptide concentrations between 1 and 10 µg/ml;

+ means significant proliferative responses obtained with peptide concentrations between 10 and 100 µg/ml;

- means no proliferative responses at any of the tested concentrations.

Fractionation experiments performed with purified populations of T and non-T cells confirmed the results in Table 1 and showed that unfractionated PBMC and purified T cells (co-cultured with autologous irradiated non-T cells as APC) express similar dose-response curves when stimulated with the core peptide analogue 50-69.

## 2. Preparation of HBV antigens

(a) A recombinant (r) preparation of HBcAg was obtained from bacterial extracts of Escherichia coli K12 strain HB 101 harboring the recombinant plasmid carrying the HBcAg coding gene as described in U.S.-A-4,710,463. Purity was approximately 90% as determined by scanning densitometry of Coomassie Blue stained SDS-polyacrylamide gel.
(b) Human cytoplasmic HBcAg (hHBcAg) was purified from the liver of a patient on dialysis as described by Petit M.A., Pillot J.J., Virol., 63:643, 1986. This core preparation had a high HBcAg activity specifically detected up to a dilution of 1:2 in solid phase radioimmunoassay with an HBeAg/HBcAg activity ratio <0.02.
(c) A HBcAg deletion mutant lacking the carboxyl-terminal 39 aminoacid residues of the core molecule (HBeAg) was produced in Escherichia coli. Purity of this antigen preparation was 99.8%. It is herein designated as rHBeAg.

## 3. Preparation of synthetic peptides

Seventeen peptides, 10-20 residues long, corresponding to the complete sequence of the core and pre-core region encoded peptides, were synthesized by Multiple Peptide System (La Jolla, CA, USA).

Peptides were designed taking into account the amphipatic regions of the antigenic molecules and the presence of structural motifs based on Rothbard algorithm. Amino acid sequences of the synthetic peptides used in this study and indicated by the aminoacid position from the $NH_2$-terminus of the core and pre-core derived polypeptides, are the following ones: 1-21, 20-34, 28-47, 38-54, 50-59, 50-64, 50-69, 70-89, 82-101, 100-119, 117-131, 120-139, 131-145, 140-155, 155-169, 169-183, 20 precore-2 core.

## 4. Isolation of peripheral blood T cells

PBMC were separated from fresh heparinized blood by Ficoll-Hypaque density gradient centrifugation. The T cells and non-T cells were isolated by rosetting PBMC with 2-aminoethylisothiuronium bromide (AET, Sigma Chemical Co., St. Louis, MO) treated sheep erythrocyte. The E-rosette forming T cells were separated front the non-rosetting (non-T) cells by Ficoll-Hypaque gradient. Purity of the T and non-T cell fractions was evaluated by direct immunofluorescence with anti-CD3+ monoclonal antibody after red cell osmotic lysis. The T cell population contained more than 95% CD3+ cells, whereas the non-rosetting fraction contained less than 3% of CD3+ cells.

Isolated cell populations were resuspended to $1\times10^5$ cells/ml in RPMI 1640 (GIBCO Laboratories, Grand Island, NY) supplemented with 25mM Hepes (GIBCO Laboratories, Grand Island, NY), 2mM L-glutamine, 50 µg/ml gentamycin and 10% human AB positive serum (complete medium).

## 5. Isolation of HBcAg-specific T cell lines

Peripheral blood T cells were cultured in round-bottomed wells of 96-well plates (Costar, Cambridge, MA) in the presence of different concentrations (0.1-1-10 µg/ml) of HBcAg, HBeAg or synthetic peptides. After 7 days, activated T cells were expanded by adding IL2 (Boehringer Mannnheim Italia S.p.A., Milan). Growing lines were restimulated after additional 5 to 7 days with the appropriate HBV antigen plus irradiated (3000 rad) autologous peripheral blood T cells as antigen presenting cells (APC) in medium supplemented with 20 U/ml of rIL2. From this point, colonies were restimulated every 7 days and provided with supplementary IL2-containing medium between restimulations to maintain the cell concentration between $3 \times 10^5$ and $1 \times 10^5$ cells/ml.

## 6. Proliferation assays

Unfractionated peripheral blood T cells ($2 \times 10^5$ cells/well) were incubated in 96 well microtiter plates (Costar, Cambridge, MA) for 7 days at 37°C in an atmosphere of 5% $CO_2$ in air in the presence of different concentrations (0.01, 0.1, 1, 10, 100 µg/ml) of each HBV antigen and synthetic peptide above described. In selected experiments, purified peripheral blood T cells ($1 \times 10^5$ cell/well) were incubated with autologous irradiated (3000 rad) non-T cells ($1 \times 10^5$ cells/well) as APC.

For the study of polyclonal T cell lines, T cells were washed extensively to remove IL2 and FCS (GIBCO Laboratories, Grand Island, NY). Subsequently, $5 \times 10^5$ cells/well were incubated for 3 days with $1 \times 10^4$ cells/well autologous irradiated (3000 rad) peripheral blood T cells as APC in complete medium in the presence of different concentrations of HBV antigens or synthetic peptides.

All proliferation assays were performed in triplicate and $^3$H-thymidine (0.5uCi/well; specific activity 2.0 Ci/mM; Amersham International, Amersham, UK) was added 18 hrs before harvesting. The results are expressed as the mean counts per minute of three replicate determinations and as the stimulation index (SI), that is the ratio between mean counts per minute obtained in the presence of antigen and mean counts per minute obtained in the absence of antigen.

a) Ten patients were studied prospectically during the symptomatic period and during convalescence for peripheral blood T cells proliferation in response to native nucleocapsid antigens, i.e. HBcAg and HbeAg as prepared in 1.-b), and synthetic peptide analogues. Time of appearance of a significant level of proliferative T cell response to the native HBcAg protein always corresponded to the time of detection of a significant T cell response to the core peptide analogue indicating that the stimulatory sequence represents the product of the nucleocapsid processing involved in the activation of core-specific T cells (Fig. 1).

b) To further investigate whether the stimulatory core peptide actually represents the fragment of the core molecule recognized by nucleocapsid-specific T cells, HBcAg and HBeAg-specific polyclonal T cell lines were produced from 5 patients by peripheral blood T cells stimulation with native antigen (see 1.-b)) and IL2. T cell lines specifically reactive to HBcAg and HBeAg, but not to other HBV antigens (envelope antigens), displayed significant levels of proliferation only in the presence of the relevant peptide recognized by parental peripheral blood T cells (Fig. 2). In the reciprocal experiment, polyclonal T cell lines produced by peripheral blood T cells stimulation with the relevant synthetic peptide were able to recognize the native core molecule (Fig. 3), confirming that the amino acid sequence represented by the synthetic peptide is actually available after processing of the native core molecule.

T cell recognition of core peptides was HLA class II restricted because peptide-induced proliferation of polyclonal T cell lines was significantly inhibited by anti-HLA class II monoclonal antibodies (anti-DP, anti-DR and anti-DQ), but not by an anti-HLA class I monoclonal antibody (Fig. 4).

## Claims

### Claims for the following Contracting States : AT, BE, CH, DE, DK, FR, GB, IT, LI, LU, NL, SE

1. A T cell stimulating polypeptide consisting of an amino acid sequence of formula (I):
PHHTALRQAILCWGELMTLA.

2. A composite polypeptide immunogen comprising the T cell stimulating polypeptide as claimed in claim 1 operatively linked to a polypeptide immunogen.

3. An immunogen according to claim 2, wherein the T cell stimulating polypeptide is operatively linked through an amino acid residue side chain to the polypeptide immunogen.

4. An immunogen according to claim 2, wherein the T cell stimulating polypeptide is operatively linked through a peptide bond to the polypeptide immunogen.

5. An immunogen according to claim 2, wherein the T cell stimulating polypeptide is operatively linked to the polypeptide immunogen by entrapment within liposomes or an immunostimulating complex.

6. An immunogen according to any one of claims 2 to 5, wherein the polypeptide immunogen comprises an antibody-inducing epitope of a virus, bacterium, fungus or protozoan.

7. An immunogen according to claim 6, wherein the epitope is an epitope of hepatitis B virus, influenza virus, foot-and-mouth disease virus, poliovirus, herpes simplex virus, rabies virus, human immunodeficiency virus or Plasmodium falciparum.

8. An immunogen according to any one of claims 2 to 5, wherein the polypeptide immunogen is HBsAg or a polypeptide comprising part of the amino acid sequence of HBsAg.

9. A process for the preparation of the T cell stimulating polypeptide claimed in claim 1, which process comprises chemically synthesising the said polypeptide or preparing the said polypeptide by recombinant DNA methodology.

10. A process for the preparation of a composite polypeptide immunogen as claimed in claim 2, which process comprises operatively linking the said T cell stimulating polypeptide to the polypeptide immunogen.

11. A vaccine comprising a pharmaceutically acceptable carrier or diluent and the T cell stimulating polypeptide claimed in claim 1 or a composite polypeptide immunogen as claimed in any one of claims 2 to 8.

**Claims for the following Contracting States : ES, GR**

1. Process for the preparation of T cell stilumating polypeptide consisting of an amino acid sequence of formula (I):

PHHTALRQAILCWGELMTLA

which process comprises chemically synthesising the said polypeptide or preparing the said polypeptide by recombinant DNA methodology.

2. A process for the preparation of a composite polypeptide immunogen comprising the T cell stimulating polypeptide as claimed in claim 1 which process comprises operatively linking the said T cell stimulating polypeptide to the polypeptide immunogen.

3. A process according to claim 2, wherein the T cell stimulating polypeptide is operatively linked through an amino acide residue side chain to the polypeptide immunogen.

4. A process according to claim 2, wherein the T cell stimulating polypeptide is operatively linked through a peptide bond to the polypeptide immunogen.

5. A process according to claim 2, wherein the T cell stimulating polypeptide is operatively linked to the polypeptide immunogen by entrapment within liposomes or an immunostimulating complex.

6. A process according to any one of claims 2 to 5, wherein the polypeptide immunogen comprises an antibody-inducing epitope of a virus, bacterium, fungus or protozoan.

7. A process according to claim 6, wherein the epitope is an epitope of hepatitis B virus, influenza virus, food-and-mouth disease virus, poliovirus, herpes simplex virus, rabies virus, human immunodeficiency virus or Plasmodium falciparum.

8. A process according to any one of claims 2 to 5, wherein the polypeptide immunogen is HBsAg or a polypeptide comprising part of the amino acid sequence of HBsAg.

9. A process for preparing a vaccine wherein a pharmaceutically acceptable carrier or diluent is admixed with the T cell stimulating polypeptide according to claim 1 or a composite polypeptide immunogen as according to any one of claims 2 to 8.

**Patentansprüche**

**Patentansprüche für folgende Vertragsstaaten : AT, BE, CH, DE, DK, FR, GB, IT, LI, LU, NL, SE**

1. T-Zellen-stimulierendes Polypeptid, bestehend aus einer Aminosäuresequenz der Formel (I):
PHHTALRQAILCWGELMTLA.

2. Zusammengesetztes Polypeptid-Immunogen, umfassend das T-Zellen-stimulierende Polypeptid nach Anspruch 1 in funktioneller Verknüpfung an ein Polypeptid-Immunogen.

3. Immunogen nach Anspruch 2, **dadurch gekennzeichnet,** daß das T-Zellen-stimulierende Polypeptid funktionell über eine Seitenkette eines Aminosäurerests an das Polypeptid-Immunogen gebunden ist.

4. Immunogen nach Anspruch 2, **dadurch gekennzeichnet,** daß das T-Zellen-stimulirende Polypeptid funktionell über eine Peptidbindung an das Polypeptid-Immunogen gebunden ist.

5. Immunogen nach Anspruch 2, **dadurch gekennzeichnet,** daß das T-Zellen-stimulierende Polypeptid funktionell an das Polypeptid-Immunogen durch Einschluß in Liposome oder einen immunstimulierenden Komplex gebunden ist.

6. Immunogen nach einem der Ansprüche 2 bis 5, **dadurch gekennzeichnet,** daß das Polypeptid-Immunogen ein Antikörper-induzierendes Epitop eines Virus, eines Bakteriums, eines Pilzes oder eines Protozoen umfaßt.

7. Immunogen nach Anspruch 6, **dadurch gekennzeichnet,** daß das Epitop ein Epitop des Hepatitis B-Virus, des Influenzavirus, des Maul-und-Klauenseuchevirus, des Poliovirus, des Herpes-simplex-Virus, des Tollwutvirus, des menschlichen Immunschwächevirus oder Plasmodium falciparum ist.

8. Immunogen nach einem der Ansprüche 2 bis 5, **dadurch gekennzeichnet,** daß das Polypeptid-Immunogen HBsAg oder ein Polypeptid, umfassend einen Teil der Aminosäuresequenz von HBsAg, ist.

9. Verfahren zur Herstellung des T-Zellen-stimulierende Polypeptids nach Anspruch 1, **dadurch gekennzeichnet,** daß man chemisch das Polypeptid synthetisiert oder das Polypeptid durch die Technik der rekombinanten DNA herstellt.

10. Verfahren zur Herstellung eines zusammengesetzten Polypeptid-Immunogens nach Anspruch 2, **dadurch gekennzeichnet,** daß man das T-Zellen-stimulierende Polypeptid funktionell an das Polypeptid-Immunogen bindet.

11. Impfstoff, umfassend einen pharmazeutisch annehmbaren Träger oder ein Verdünnungsmittel und das T-Zellen-stimulierende Polypeptid nach Anspruch 1 oder ein zusammengesetztes Polypeptid-Immunogen nach einem der Ansprüche 2 bis 8.

**Patentansprüche für folgende Vertragsstaaten : ES, GR**

1. Verfahren zur Herstellung eines T-Zellen-stimulierende Polypeptids, bestehend aus einer Aminosäuresequenz der Formel (I):
PHHTALRQAILCWGELMTLA,
**dadurch gekennzeichnet,** daß man chemisch das Polypeptid synthetisiert oder das Polypeptid durch die Technik der rekombinaten DNA herstellt.

2. Verfahren zur Herstellung eines zusammengesetzten Polypeptid-Immunogens, umfassend das T-Zellen-stimulierende Polypeptid nach Anspruch 1, **dadurch gekennzeichnet,** daß man das T-Zellen-stimulierende Polypeptid funktionell mit dem Polypeptid-Immunogen verbindet.

3. Verfahren nach Anspruch 2, **dadurch gekennzeichnet,** daß das T-Zellen-stimulierende Polypeptid funktionell über eine Seitenkette eines Aminosäurerests an das Polypeptid-Immunogen gebunden ist.

4. Verfahren nach Anspruch 2, **dadurch gekennzeichnet,** daß T-Zellen-stimulierende Polypeptid funktionell über eine Peptidbindung an das Polypeptid-Immunogen gebunden ist.

5. Verfahren nach Anspruch 2, **dadurch gekennzeichnet,** daß das T-Zellen-stimulierende Polypeptid funktionell an das Polypeptid-Immunogen durch Einschluß in Liposome oder einen immunstimulierenden Komplex gebunden ist.

6. Verfahren nach einem der Ansprüche 2 bis 5, **dadurch gekennzeichnet,** daß das Polypeptid-Immunogen ein Antikörper-induzierendes Epitop eines Virus, eines Bakteriums, eines Pilzes oder eines Protozoen umfaßt.

7. Verfahren nach Anspruch 6, **dadurch gekennzeichnet,** daß das Epitop ein Epitop des Hepatitis B-Virus, des Influenzavirus, des Maul-und-Klauenseuchevirus, des Poliovirus, des Herpes-simplex-Virus, des Tollwutvirus, des menschlichen Immunschwächevirus oder Plasmodium falciparum ist.

8. Verfahren nach einem der Ansprüche 2 bis 5, **dadurch gekennzeichnet,** daß das Polypeptid-Immunogen HBsAg oder ein Polypeptid, umfassend einen Teil der Aminosäuresequenz von HBsAg ist.

9. Verfahren zur Herstellung eines Impfstoffs, **dadurch gekennzeichnet,** daß ein pharmazeutisch annehmbarer Träger oder ein Verdünnungsmittel mit dem T-Zellen-stimulierenden Polypeptid nach Anspruch 1 oder einem zusammengesetzten Polypeptid-Immunogen nach einem der Ansprüche 2 bis 8 vermischt wird.

## Revendications

**Revendications pour les Etats contractants suivants : AT, BE, CH, DE, DK, FR, GB, IT, LI, LU, NL, SE**

1. Polypeptide stimulant les cellules T, constitué d'une d'une séquence d'acides aminés répondant à la formule (1) :

PHHTALRQAILCWGELMTLA.

2. Immunogène polypeptidique composite comprenant le polypeptide stimulant les cellules T selon la revendication 1, lié par liaison opérative à un immunogène polypeptidique.

3. Immunogène selon la revendication 2, dans lequel le polypeptide stimulant les cellules T est lié par liaison opérative, par l'intermédiaire d'une chaine latérale de résidus d'acides aminés, à l'immunogène polypeptidique.

4. Immunogène selon la revendication 2, dans lequel le polypeptide stimulant les cellules T est lié par liaison opérative à l'immunogène polypeptidique par l'intermédiaire d'une liaison peptidique.

5. Immunogène selon la revendication 2, dans lequel le polypeptide stimulant les cellules T est lié par liaison opérative à l'immunogène polypeptidique par piégeage entre les liposomes ou un complexe immuno-stimulant.

6. Immunogène sleon l'une quelconque des revendications 2 à 5, dans lequel l'immunogène polypeptidique comprend un épitope produisant un anticorps, d'un virus, d'une bactérie, d'un champignon ou d'un protozoaire.

7. Immunogène selon la revendication 6, dans lequel l'épitope est un épitope du virus de l'hépatite B, du virus de la grippe, du virus de la fièvre aphteuse, du virus de la polyomyélite, d'un virus herpes simplex, du virus de la rage, du virus de l'immunodéficience humaine ou de Plasodium falciparum.

8. Immunogène selon l'une quelconque des revendications 2 à 5, dans lequel l'immunogène polypeptidique est HBsAg ou un polypeptide comprenant une partie de la séquence des acides aminés du HBsAg.

9. Procédé pour préparer le polypeptide stimulant les cellules T selon la revendication 1, procédé consistant à synthétiser par voie chimique ledit polypeptide ou à préparer ledit polypeptide par la technique de l'ADN recombinant.

10. Procédé pour préparer un immunogène polypeptidique composite selon la revendication 2, lequel procédé consiste à lier par liaison opérative à l'immunogène polypeptidique ledit polypeptide stimulant les cellules T.

11. Vaccin comprenant un excipient ou un diluant pharmaceutiquement acceptable et le polypeptide stimulant les cellules T selon la revendication 1 ou un immunogène polypeptidique composite selon l'une quelconque des revendicatioons 2 à 8.

**Revendications pour les Etats contractants suivants : ES, GR**

1. Procédé pour préparer un polypeptide stimulant les cellules T, constitué d'une séquence d'acides aminés répondant à la formule (I)

PHHTALRQAILCWGELMTLA

lequel procédé consiste à synthétiser par voie chimique ledit polypeptide ou à préparer ledit polypeptide par la technique de l'ADN recombinant.

2. Procédé pour préparer un imunogène polypeptidique composite comprenant le polypeptide stimulant les cellules T selon la revendication 1, lequel procédé consiste à lier par liaison opérative ledit polypeptide stimulant les cellules T à l'immunogène polypeptidique.

3. procédé selon la revendication 2, dans lequel le polypeptide stimulant les cellules T est lié par liaison opérative, par l'intermédiaire d'une chaine latérale de résidus d'acides aminés, à l'immunogène polypeptidique.

4. Procédé selon la revendication 2, dans lequel le polypeptide stimulant les cellules T est lié par liaison opérative à l'immunogène polypeptidique par l'intermédiaire d'une liaison peptidique.

5. Procédé selon la revendication 2, dans lequel le polypeptide stimulant les cellules T est lié par liaison opérative à l'immunogène polypeptidique par piégeage entre les liposomes ou un complexe immuno-stimulant.

6. Procédé sleon l'une quelconque des revendications 2 à 5, dans lequel l'immunogène polypeptidique comprend un épitope produisant un anticorps, d'un virus, d'une bactérie, d'un champignon ou d'un protozoaire.

7. Procédé selon la revendication 6, dans lequel l'épitope est un épitope du virus de l'hépatite B, du virus de la grippe, du virus de la fièvre aphteuse, du virus de la polyomyélite, d'un virus herpes simplex, du virus de la rage, du virus de l'immunodéficience humaine ou de Plasmodium falciparum.

8. Procédé selon l'une quelconque des revendications 2 à 5, dans lequel l'immunogène polypeptidique est HBsAg ou un polypeptide comprenant une partie de la séquence des acides aminés du HBsAg.

9. Procédé pour préparer un vaccin, dans lequel on mélange un excipient ou un diluant pharmaceutiquement acceptable au polypeptide stimulant les cellules T selon la revendication 1 ou à un immunogène polypeptidique composite selon l'une quelconque des revendications 2 à 8.

Fig. 1

Legend:
- HBcA
- pep. 1-20
- pep. 50-69
- pep. 117-131

Fig. 2

Legend:
- HBcA
- pep. 1-20
- pep. 50-69
- pep. 117-131

Fig. 3

Legend:
- HBcA
- pep. 1-20
- pep. 50-69
- pep. 117-131

Fig. 4

Fig. 5

Fig. 6

15

Medium
HBcAg
pep.20-34
pep.50-69

Fig. 7

+anti-DR    +anti-DP    +anti-DQ    +anti-class I

Fig. 8